# FASCICULE DE BREVET EUROPEEN

(11) **EP 2 874 661 B1**
(45) Date de publication et mention de la délivrance du brevet: **08.08.2018**
(21) Numéro de dépôt: 13747451.6
(22) Date de dépôt: 17.07.2013
(51) Int. Cl.: A61K 47/18, A61K 9/107

(54) **UTILISATION D'UNE CREME DE PROTECTION CONTRE LES EFFETS DES AGENTS CHIMIQUES AGRESSIFS EN CONTACT AVEC LA PEAU**
VERWENDUNG EINER SALBE ZUM SCHUTZ GEGEN DIE WIRKUNG AGGRESSIVER CHEMISCHER MITTEL IN KONTAKT MIT DER HAUT
USE OF A CREAM THAT PROTECTS AGAINST THE EFFECTS OF AGGRESSIVE CHEMICAL AGENTS IN CONTACT WITH THE SKIN

(30) Priorité: 18.07.2012 FR 1256939
(43) Date de publication de la demande: 27.05.2015
(73) Titulaire: PREVOR INTERNATIONAL, 75015 Paris (FR)
(72) Inventeur: BLOMET, Joël, F-95760 Valmondois (FR); MATHIEU, Laurence, F-33400 Talence (FR); MEYER, Marie-Claude, F-75015 Paris (FR)
(74) Mandataire: Cabinet Plasseraud
(86) Numéro de dépôt international: PCT/FR2013/051717
(87) Numéro de publication internationale: WO 2014/013194

(56) Documents cités:
- WO-A2-01/93858
- FR-A1- 2 604 900
- US-A- 5 763 486

## Description

### Domaine de l'invention

La présente invention se rapporte au domaine des produits de protection contre des agents chimiques agressifs qui peuvent entrer en contact avec la peau.

### Contexte de l'invention

Lors de la manipulation de produits chimiques agressifs, notamment dans les usines, les laboratoires, les salons de coiffure, etc., il est fréquent que des projections accidentelles de ces produits entrent en contact avec la peau. De 10% à 15% des maladies professionnelles sont des maladies de peau, 95% de celles-ci étant des dermites de contact. La dermite de contact professionnelle concerne principalement les mains. On observe de 0,7 à 1,5 cas pour 1 000 travailleurs par an.

La fréquence des irritations de la peau est fonction tant de la susceptibilité individuelle que de la fréquence et de la durée d'exposition aux agents chimiques agressifs. Des facteurs importants sont par exemple le travail en ambiance humide, avec des détergents, des acides, des bases, des oxydants, des solvants, etc. En outre, il faut souligner que les dermites d'irritation chroniques constituent également un facteur de risque important pour des sensibilisations secondaires.

Afin de réduire le risque de développer des dermatoses professionnelles, le port de gants est fortement recommandé. Cependant, le port des gants prescrits n'est pas toujours effectif pendant 100 % du temps de travail. En effet, il arrive très fréquemment que le port de gants ne soit pas totalement respecté notamment en cas de manipulation d'agents modérément agressifs car il est jugé trop contraignant par l'utilisateur. Les manipulateurs ont alors tendance à limiter la durée de port des gants. Par ailleurs, l'efficacité de la protection peut n'être que partielle si la matière du gant n'est pas parfaitement adaptée au produit chimique contre lequel on veut se protéger et/ou si la durée de port est prolongée, dépassant le temps de perméation. De plus, des infiltrations des agents chimiques agressifs peuvent avoir lieu soit en raison d'une déchirure du gant ou en raison du fait que des gouttes d'agent chimique agressif peuvent entrer en contact avec la peau par la manchette du gant ou avant ou après le port des gants. L'application d'une crème destinée à construire une barrière physique contre certaines substances spécifiques qui sont légèrement agressives ou certains allergènes spécifiques, permet de protéger la peau contre ces agents notamment lorsque ceux-ci entrent en contact avec la peau avant ou après le port des gants.

Au cours des trois dernières décennies, la préparation de telles crèmes de protection est passée du stade empirique au stade de recherche scientifique. Les formulations protectrices pour la peau (crème et gel) sont à base de divers véhicules topiques (en général des émulsions huile-dans-eau ou eau-dans-huile et également des gels à base d'éther de cellulose ou de polymère acrylique). Cependant, de telles formulations sont toujours conçues pour une utilisation dans des conditions environnementales déterminées, c'est-à-dire pour protéger contre un type d'agents irritants spécifique.

Ainsi, dans le commerce il existe de telles crèmes/gels. Le problème qui se pose est que ces formulations sont destinées à la protection de la peau seulement contre un spectre étroit des agents légèrement agressifs et solubles dans l'eau ou dans l'huile. Cela est lié au fait que leur mode de fonctionnement repose sur un effet mécanique: une fois pénétrée la crème forme une barrière entre la peau et les agents agressifs extérieurs. Cette barrière est efficace à l'encontre des agents agressifs qui n'y sont pas solubles. Ainsi, l'efficacité de tels produits est réduite puisque seule la solubilité est prise en considération et non d'autres paramètres tels que le poids moléculaire, le coefficient de partage, la réactivité chimique, etc. De tels produits ne sont jamais d'utilisation universelle.

US 5,763,486 A décrit une composition physiologique pour traiter les brûlures thermiques et chimiques.

WO 01/93858 A2 décrit l'utilisation d'un complexe formé entre l'ion Al³⁺ et le ligand EDTA pour la fabrication d'un produit destiné à la prévention et/ou au traitement des urticaires provoquées par des agents urticants choisis dans le groupe comprenant les méduses, les orties, le mancellinier, le sumac vénéneux et les piqûres d'insectes.

Actuellement il n'existe aucune crème capable de protéger efficacement la peau contre un large spectre d'agents chimiques de toute nature, et notamment contre les allergènes.

De façon surprenante et inattendue les présents inventeurs ont formulé une crème permettant de protéger la peau contre tout type d'agents chimiques agressifs.

### Résumé de l'invention

D'une façon générale, la présente invention porte sur l'utilisation d'une crème ou émulsion pour la protection de la peau contre les agents chimiques agressifs ladite crème comprenant un agent chélatant amphotère. Elle porte également sur ladite émulsion. Au sens de la présente invention, par émulsion on entend une émulsion choisie dans le groupe comprenant une émulsion huile dans l'eau, une émulsion eau dans l'huile, une émulsion multiple, une microémulsion, une nano-émulsion, et leurs mélanges.

Plus spécifiquement, selon un premier aspect, la présente invention porte sur une émulsion pour son utilisation pour protéger la peau contre les agents chimiques agressifs, et notamment contre les allergènes.

Selon un deuxième aspect, la présente invention porte sur ladite émulsion pour son utilisation pour la prévention des dermites de contact.

Il est également décrit le procédé de préparation de ladite émulsion.

Une description plus détaillée de certains modes de réalisation préférés de l'invention est donnée ci-dessous.

### Description Détaillée de l'Invention

La présente invention porte sur une émulsion pour son utilisation pour la protection de la peau contre les agents chimiques agressifs telle que revendiquée dans la revendication 1, ladite émulsion comprenant au moins un agent chélatant amphotère comprenant un complexe à base d'aluminium et d'acide éthylène diamine tétraacétique déprotoné, présentant la formule générale [Al(Y)Bₙ]^{c'}D_{c} avec B représentant OH⁻, BO₂⁻, H⁺, Y représentant un tétracarboxylate pouvant être protoné quatre fois pour former l'acide éthylène diamine tétraacétique, n représentant un nombre entier égal à 0 , 1, 2 ou 3, D étant un contre-ion, de préférence Na⁺, c étant un nombre entier égal à 0, 1, 2 ou 3 et c' étant un nombre relatif ayant la même valeur absolue que c.

Cet agent chélatant amphotère peut être formé de préférence par une combinaison quasi-stoechiométrique de l'ion aluminium Al³⁺, du ligand Y et d'un agent stabilisant choisi parmi OH⁻, BO₂⁻ ou H⁺. En conséquence, son pH reste neutre, le plus petit de ses pK acides est dans la gamme de 6 à 10 alors que le plus grand de ses pK basiques est dans la gamme de 5 à 8, et le plus grand pK basique est inférieur au plus petit pK acide.

Au sens de la présente invention, on entend par crème une émulsion, c'est-à-dire, une émulsion huile dans l'eau, une émulsion eau dans huile, une émulsion multiple, une microémulsion, ou une nano-émulsion. Ces deux termes "crème" et "émulsion" pourront donc être utilisés indifféremment dans la présente description.

Selon un mode de réalisation particulier, ledit complexe à base d'aluminium et d'Y est stabilisé par une base faible telle que les acides aminés choisis dans le groupe comprenant la glycine, l'histidine, l'arginine, la lysine, la phénylalanine, l'alanine, l'isoleucine, la leucine, la méthionine, la proline, la valine, le tryptophane, la sérine, la glutamine, la cystine et leurs mélanges. La glycine est tout à fait appropriée.

La quantité d'agent chélatant amphotère présente dans ladite émulsion est de 0,01 à 5%, de préférence de 0,1 à 2% et plus préférentiellement encore de 0,2 à 1% en poids du poids total de l'émulsion. Une quantité inférieure à 0,01% ne permet pas d'obtenir un effet de protection suffisant. Au-delà de 5% on n'observe pas d'amélioration sensible.

Selon l'invention, l'émulsion est utilisée en application topique, plus particulièrement cutanée.

Sans vouloir être liés par aucune théorie, les présents inventeurs sont d'avis que l'émulsion selon l'invention permet de réaliser une barrière physique et chimique contre les agents chimiques agressifs. Plus particulièrement, après application et séchage, une couche barrière dépourvue d'eau recouvre la peau assurant ainsi une barrière physique entre la peau et l'environnement extérieur, l'agent chélatant amphotère assurant quant à lui une barrière chimique en chélatant ou en réagissant chimiquement avec les agents chimiques entrant en contact avec cette couche de crème.

Par cette double action, les agents agressifs sont empêchés d'entrer en contact avec la peau.

Pour être efficace, l'émulsion doit recouvrir en totalité la zone de peau à protéger, généralement les mains. Elle doit être appliquée régulièrement sur toute la surface, à raison d'environ 0,5 à 5 mg/cm², de préférence d'environ 1 à 3 mg/cm², et plus préférentiellement encore d'environ 2 mg/cm².

Pour une efficacité optimale, le produit est appliqué au moins 5 minutes, de préférence au moins 10 minutes et plus préférentiellement encore au moins 15 minutes avant le contact avec des agents chimiques agressifs.

En fonction de l'activité, la couche protectrice formée sur la peau sera plus ou moins durable. En tout état de cause, l'application de la crème selon l'invention devra être renouvelée après chaque lavage de mains. En l'absence de lavage de mains et de frottements mécaniques importants et/ou répétés, la couche protectrice pourra être efficace jusqu'à 4 heures. Si l'activité engendre de nombreux frottements, il conviendra de renouveler l'application plus fréquemment. Ainsi, il est recommandé de renouveler l'application de l'émulsion selon l'invention au moins toutes les 4 heures ou après chaque lavage des mains.

Au sens de la présente invention, les agents chimiques agressifs regroupent des produits chimiques irritants et des produits allergènes. Les produits chimiques irritants comprennent des substances et des mélanges qui appartiennent aux catégories H311, H312, H314, H315, H317 telles que définies dans le règlement CLP (règlement (CE) n°1272/2008 relatif à la classification, à l'étiquetage et à l'emballage des substances et des mélanges). Ils incluent également les produits ayant un pH de 2 à 11,5.

Ainsi, dans le règlement CE n° 1272/2008, le produit H311 est défini comme toxique par contact cutané. Le produit H312 est défini comme nocif par contact cutané. Le produit H314 est défini comme provoquant de graves brûlures de la peau et des lésions oculaires. Le produit H315 est défini comme provoquant une irritation cutanée. Le produit H317 est défini comme pouvant provoquer une allergie cutanée.

L'émulsion selon l'invention satisfait la norme NFS75601. Cette norme concerne les crèmes protectrices qui s'appliquent préventivement sur la peau pour assurer une protection temporaire contre les produits modérément agressifs. On entend par produits modérément agressifs les substances ou préparations qui ne sont pas classées très toxiques, toxiques, corrosives ou allergisantes par la réglementation relative à l'étiquetage des substances dangereuses. Cette norme fixe les caractéristiques de ces crèmes protectrices et leurs limites d'emploi. Elle ne s'applique pas aux préparations destinées à protéger éventuellement contre les risques physiques ou microbiologiques.

L'émulsion peut être utilisée de façon efficace contre les produits chimiques irritants hydrosolubles ou non hydrosolubles.

A titre d'exemples de produits irritants hydrosolubles, on peut citer les acides, les bases, les produits chlorés, etc. Plus particulièrement, ces produits peuvent être choisis dans le groupe comprenant l'hydroxyde de sodium de pH ≤ 11,50, l'acide chlorhydrique de pH ≥ 2,00, l'hypochlorite de sodium jusqu'à 12%, le laurylsulfate de sodium en solution aqueuse à une concentration pouvant aller jusqu'à 5% et leurs mélanges. A titre d'exemple de produits irritants non hydrosolubles, on peut citer: les solvants hydrocarbonés à longue chaîne, les solvants chlorés et leurs mélanges. En particulier, de tels produits peuvent être choisis dans le groupe comprenant l'huile minérale, le White spirit (C9-C16 Alkane/cycloalkane) et leurs mélanges. On peut également citer les produits d'usage courant tels que les détergents, colles, décapants, produits de traitement du bois, les produits de traitement agricoles tels que insecticides, pesticides, engrais ; les ciments et mortiers, les produits de comblement ou solidification des cavités rocheuses, les teintures. La crème selon l'invention est efficace contre les produits nécessitant un étiquetage spécifique en raison de leur toxicité.

Les produits allergènes comprennent des substances, particules ou corps organiques (atomes, molécules, protéines, glycoprotéines ou toute autre substance macromoléculaire organique de structure complexe), qui, en contact avec la peau, sont capables de provoquer une réaction allergique chez un sujet préalablement sensibilisé. Cette réaction allergique peut entraîner un dommage à la peau ou même un phénomène pathologique. A titre d'exemples de produits allergènes, on peut citer les allergènes choisis dans le groupe comprenant des métaux lourds (par exemple : Al, As, Cd, Cr, Hg, Ni, Pb, Sr, Te), le disulfure de tétraméthylthiuram, le 2-mercaptobenzothiazole, le carbamate de benzyle, le bisphénol, l'acrylate d'éthyle, l'araldite 506 epoxy Resin, l'hévéine, le méthanal, les allergènes de type protéinique (tels que les profilines, les tropomyosines, les LTP (les protéines de transfert de lipides), les bet v 1-like protéines PR-10, les polcalcines, les bêta parvalbumines, les 2s albumines, les bêta expansines, les polygalacturonases, les Ag5 (antigènes 5), les albumines, les caséines, les lipocalines, le groupe 5 des graminées, les 11s globulines, les 7s vicilin-like globulines, le groupe 4 des graminées, les papaïne-like protéases à cystéine, les phospholipases A1, les inhibiteurs de protéase à sérine, les hyaluronidases, les chitinases de classe 1, les thaumatin-like protéines, etc.) et leurs mélanges.

L'efficacité d'utilisation de l'émulsion conforme à l'invention a été testée par des analyses morphologiques.

Pour prouver l'efficacité contre des irritants, deux méthodologies ont été mises en oeuvre : des analyses de morphologie et des études permettant le dosage des interleukines et notamment des IL-6 et IL-8.

L'analyse de morphologie cellulaire a été réalisée par une coloration au trichrome de Masson, variante Goldner. La présence de modifications morphologiques signale que la substance testée a suscité une irritation forte qui a ainsi modifié la structure de cellules. Après application de l'émulsion huile dans l'eau selon l'invention, aucune modification morphologique n'a été observée après mise en contact de la zone de peau couverte par l'émulsion par les agents chimiques agressifs suivants: l'hydroxyde de sodium à pH 11,50, l'acide chlorhydrique à pH 2,00, l'hypochlorite de sodium à 12%, le laurylsulfate de sodium à 5%, l'huile minérale et le White spirit (C9-C16 Alkane/cycloalkane).

L'efficacité a aussi été démontrée par une expérimentation avec dosage des interleukines.. La concentration d'interleukine IL-6 (produit en premier temps) et IL-8 (produit en second temps) dans le derme est fonction du processus d'inflammation de la peau. Après application de l'émulsion sur la peau, il n'a pas été observé d'augmentation sensible de la teneur en interleukine 6 ou 8 après mise en contact de la zone de peau couverte par l'émulsion avec les agents suivants: l'hydroxyde de sodium à pH 11,50, l'acide chlorhydrique à pH 2,00, l'hypochlorite de sodium à 12%, le laurylsulfate de sodium à 5%, l'huile minérale, et le White spirit (C9-C16 Alkane/cycloalkane).

L'efficacité d'utilisation de l'agent chélatant amphotère conforme à l'invention a été testée par des analyses morphologiques basées sur la théorie suivante : Les cellules de Langerhans sont des cellules dendritiques que l'on retrouve dans l'épiderme et qui contiennent des granules de Birbeck. Ils existent normalement dans des ganglions lymphatiques et dans la peau au niveau du stratum spinosum de l'épiderme. Ces cellules spécialisées dans la captation des antigènes s'activent lorsque la peau est mise en contact avec un allergène. Cela présente un premier signe du début d'un processus de sensibilisation. Des méthodes d'immuno-marquage, notamment des récepteurs de surface CD1a des cellules de Langerhans permettent d'en mesurer le nombre et d'observer leur migration de l'épiderme au derme. La composition préventive de l'invention est efficace si l'antigène est stoppé, c'est-à-dire non capté par les cellules de Langerhans épidermiques. Celles-ci ne vont donc pas migrer vers le derme. L'efficacité de la composition selon l'invention se mesure donc par le nombre de cellules de Langerhans non migrantes par centimètre d'épiderme. Il n'a pas été observé de migration sensible du nombre de ces cellules après mise en contact de la zone de peau couverte par l'émulsion avec des métaux lourds (par exemple : Al, As, Cd, Cr, Hg, Ni, Pb, Sr, Te), disulfure de tétraméthylthiuram, le 2-mercaptobenzothiazole, le carbamate de benzyle, le bisphénol, l'acrylate d'éthyle, l'araldite 506 epoxy Resin, l'hévéine et le méthanal.

Selon un autre aspect, la présente invention porte sur une émulsion telle que revendiquée dans la revendication 5 pour une utilisation dans la prévention des dermites de contact.

La dermite de contact est une réaction cutanée résultant de l'exposition prolongée ou répétée à des substances allergènes ou irritantes. On distingue dermites de contact irritatives ou DCI et dermites de contact allergiques ou DCA.

Parmi les dermites de contact allergiques on distingue : les réactions de type immédiates : urticaire de contact et les réactions de type retardées : eczéma.

Par ailleurs, les dermites irritatives peuvent présenter un caractère aigu ou chronique.

Par « dermites de contact » au sens de l'invention, on entend tous ces types de réactions cutanées.

L'émulsion selon l'invention empêche le contact direct de la peau avec lesdits agents et ainsi aide à éviter les dermites de contact par construction d'une barrière physique en formant un film dépourvu d'eau et également une barrière chimique utilisant à la fois les propriétés d'agent chélatant amphotère et les propriétés réactives de la crème selon l'invention.

Ainsi, l'émulsion permet une prévention des dermites de contact, provoquées par certains agents chimiques ou même par le port des gants de protection.

L'émulsion selon l'invention peut être appliquée quotidiennement. Les conditions d'utilisation sont identiques à celles décrites précédemment.

Les modes de réalisation décrits ci-après concernent à la fois l'émulsion en tant que telle et l'utilisation de ladite émulsion.

Selon un mode de réalisation particulier, ladite émulsion est une émulsion huile dans l'eau.

L'émulsion comprend une phase aqueuse, une phase huileuse et au moins un agent tensioactif.

L'agent tensio-actif peut être hydrophobe avec un HLB de 3 à 10 (pour les émulsions qui ne sont pas huile dans eau) ou hydrophile avec un HLB de 11 à 18. Cet agent tensio-actif est choisi dans le groupe comprenant des alcools gras éthoxylés, des acides et des esters gras (par exemple : cétéareth-12, cétéareth-20, cétéareth-33, stéaryl cétyl alcool 20-éthoxylé, 2-polyhydroxystéarate polyglycéryl, glycéryl oléate, ester de sorbitan, ester de glycérol, PEG-mono/di-laurate, PEG-mono/di-stéarate, cétéaryl isononanoate, glycéryl stéarate, etc.), des carboxylates, des éthoxycarboxylates (par exemple : stéarate de sodium/potassium, acide alkyl-carboxylique, acide carboxylique éther alkyl-polyglycol, acide carboxylique éther polyglycol alkylphénol, alcool carboxyméthylé, éthoxycarboxylate, éthercarboxylate, etc.) et leurs mélanges.

La quantité d'agent tensio-actif est de 0,1% à 10% en poids, de préférence de 0,5 à 5% en poids, et plus préférentiellement encore de 1 à 3% en poids du poids total de l'émulsion.

La phase huileuse comprend des cires ou huiles synthétiques ou naturelles, choisies dans le groupe comprenant extrait de Carnauba, cire d'abeille, beurre de karité, triglycérides, stéarines, esters d'acides gras (par exemple : des alcools cétéaryliques, des éthers dicapryliques de cétyl palmitate, des carbonates dicapryliques, des isononanates cétéaryliques, des dimères distéaryl-tricarbonate, etc.), des huiles de silicone, des stéarates de zinc, des polyisobutènes, des octyldodécanols, des octyldécylxylosides, des alcools gras, des acides gras (par exemple : acide laurique, acide myristique, acide stéarique, etc.), des huiles végétales (par exemple: huile de tournesol, de jojoba, de cocos nucifera, de soja, d'amande, etc.) et leurs mélanges.

La phase huileuse représente jusqu'à 98% en poids, notamment jusqu'à 70% en poids, de préférence 5 à 50% en poids, et plus préférentiellement encore de 10 à 20% en poids du poids total de l'émulsion.

La phase aqueuse comprend essentiellement de l'eau, de préférence de l'eau purifiée. Elle est présente à raison de 1 à 90% en poids, de préférence de 30 à 80% en poids, et plus préférentiellement encore de 50 à 70% en poids du poids total de l'émulsion.

L'émulsion selon l'invention peut inclure divers agents conférant des propriétés avantageuses pour la peau. Ces agents, selon leur solubilité sont présents ou bien dans la phase aqueuse ou bien dans la phase huileuse.

Ces agents sont notamment des agents hydratants et émollients, des agents anti-perspirants, etc.

Les agents hydratants et émollients peuvent être choisis dans le groupe comprenant allantoïne, polyol (par exemple glycérol, polymères de glycérol, propylène glycol, sorbitol etc.), des extraits végétaux (par exemple des extraits d'aloe vera, de camomille, de concombre, de calendula, etc.), acide hyaluronique, acide pyrrolidone carboxylique, urée, chitosan, tocophérol, panthénol, butylène glycol, phospholipide, acide linoléique, acide γ-linoléique, alpha-bisabolol, et leurs mélanges.

La concentration en agents hydratants et émollients est de 0,1 à 20% en poids, de préférence de 0,5 à 10% en poids, et plus préférentiellement encore de 1 à 5% en poids du poids total de l'émulsion.

Les agents anti-perspirants peuvent être choisis dans le groupe comprenant des sels d'aluminium, de préférence le sesqui-chlorhydrate d'aluminium, des sels d'aluminium et de zirconium, des complexes d'aluminium-zirconium octachlorohydrex glycine et leurs mélanges. La concentration en agent anti-perspirant est de 0,1 à 50% en poids, de préférence de 10 à 30% en poids, et plus préférentiellement encore de 15% en poids du poids total de l'émulsion.

L'émulsion peut également comprendre des additifs améliorant la texture tels que des conditionneurs et des épaississants.

Les conditionneurs peuvent être choisis dans le groupe comprenant des polymères polycationiques désignés selon la nomenclature INCI polyquaterniums, des gommes quaternisées, des phospholipides quaternisés, et leurs mélanges. La concentration en conditionneur est de 0,1 à 20% en poids, de préférence de 0,5 à 10% en poids, et plus préférentiellement encore de 0,5 à 5% en poids du poids total de l'émulsion.

Les épaississants peuvent être choisis dans le groupe comprenant : des polymères et des copolymères d'acide acrylique, des polymères réticulés acrylate d'alkyle en C₁₀-₃₀/acrylate, polyacrylamide, poloxamer, dérivés cellulosiques (esters et éthers), silices, fumée de silice, silicates,tels que silicates de magnésium-aluminium, chitine et ses dérivés, gélatine, xanthane, dextrane, gellane, carraghénanes, alginates, agar-agar, gélose, pectine, gomme d'acacia, gomme karaya, gomme adragante, gomme arabique, gomme de guar, gomme de caroube, amidon et ses dérivés, scléroglucane et leurs mélanges. La concentration en épaississant est de 0,01 à 10% en poids, de préférence de 0,1 à 5% en poids, et plus préférentiellement encore de 0,5 à 3% en poids du poids total de l'émulsion.

L'émulsion peut également comprendre des conservateurs. Ceux-ci peuvent être choisis dans le groupe comprenant: ester para-hydroxybenzoique, isothiazolinone, imidazolidinyl urée, diazolidinyl urée, des bromo-nitro-propanediol, phénoxyéthanol, acide sorbique et ses sels, acide benzoique et ses sels, phénoxyéthanol, alcool benzylique, et leurs mélanges. La concentration est celle autorisée en cosmétique.

Enfin, la formulation peut comprendre des colorants cosmétiques et alimentaires, des parfums, des arômes, des agents de régulation du pH (par exemple, acide citrique, acide lactique, acide phosphorique, hydroxyde de sodium, potasse, amino-méthyl-propanol, triéthanolamine, etc.) et leurs mélanges. Leur concentration peut être de 0,1 à 50% en poids, de préférence de 10 à 30% en poids, et plus préférentiellement encore de 15% en poids du poids total de l'émulsion.

Il est également décrit le procédé de préparation de ladite émulsion.

Ladite émulsion peut être préparée selon toute technique connue de l'homme du métier. Cependant, afin d'optimiser sa stabilité, il convient de préparer dans un premier temps la phase aqueuse en y incorporant l'agent chélatant amphotère par mélange à froid puis d'incorporer cette préparation dans la phase huileuse avant de mélanger en vue de l'émulsification.

Dans un deuxième mode de réalisation, il convient de préparer la phase aqueuse en y incorporant l'agent chélatant amphotère par mélange à chaud, puis d'incorporer cette préparation dans la phase huileuse avant de mélanger en vue de l'émulsification.

Le mélange à chaud peut se faire à 50-90°C, de préférence à 60-80°C et plus préférentiellement encore à 70°C.

Au sens de la présente invention, on considère que la stabilité est satisfaisante si on n'observe pas de déphasage après stockage de l'émulsion à la température ambiante pendant 6 mois, de préférence pendant 1 an.

La présente invention va être décrite de façon plus détaillée à l'aide des exemples suivants.

### EXEMPLES

Les exemples suivants décrivent certains modes de réalisation de la présente invention. Cependant, il est entendu que les exemples ne sont présentés qu'à titre illustratif seulement et ne limitent en aucun cas la portée de l'invention.

Dans les présents exemples, l'agent chélatant amphotère utilisé présente la formule suivante: [AlYBO₂]²⁻Na⁺₂.

### EXEMPLE 1 : EMULSION POUR USAGE EXTERNE

On a préparé une émulsion comprenant les produits suivants :

| | |
|---|---|
| Stéarate de glycérol / Alcool cétéarylique/ cétyl palmitate/ Glycérides de coco (ratio en poids: 60%/20%/10%/10%) | 0,12g |
| Alcool cétéarylique éthoxylé (12 moles) | 0,01g |
| Alcool cétéarylique éthoxylé (20 moles) | 0,02g |
| C18- caprylate/ Caprate | 0,09g |
| Caprylique/ caprique triglycéride | 0,03g |
| Paraffine liquide | 0,02g |
| Propylparaben/ benzoate paraben (50/50 p/p) | 0,004g |
| Eau purifiée | qsp 1g |
| Glycérine | 0,03g |
| Agent chélatant amphotère | 0,002g |

Cette émulsion huile dans l'eau présente les caractéristiques suivantes:
- viscosité Brookfield de 18000 cP, mesurée à une température de 20(+/-1)°C et avec une vitesse de mobile à 3/20 rpm,
- pH de 6,45,
- Aucun déphasage ne s'est produit après stockage pendant 6 mois à la température ambiante.

### EXEMPLE 2 : EMULSION POUR USAGE EXTERNE

De la même manière que dans l'exemple 1, on a préparé une émulsion contenant:

| | |
|---|---|
| Stéarate de glycérol / Alcool cétéarylique/ cétyl palmitate/ Glycérides de coco (ratio en poids 60%/20%/10%/10%) | 0,12g |
| Alcool cétéarylique éthoxylé (12 moles) | 0,011g |
| Alcool cétéarylique éthoxylé (20 moles) | 0,022g |
| C18- caprylate/ Caprate | 0,08g |
| Caprylique/ caprique triglycéride | 0,04g |
| Dicaprylyl éther | 0,03g |
| Paraffine liquide | 0,025g |
| Oxyde de zinc | 0,01g |
| Propylparabène, benzoate parabène (50/50 p/p) | 0,004g |
| Eau purifiée | qsp 1g |
| Glycérine | 0,03g |
| Acide citrique | 0,001g |
| Silicate d'aluminium et de magnésium | 0,007g |
| Agent chélatant amphotère | 0,002g |

Cette émulsion huile dans l'eau présente les caractéristiques suivantes:
- viscosité Brookfield de 21000 cP, mesurée à une température de 20(+/-1)°C et avec une vitesse de mobile à 3/20 rpm
- pH de 6,55
- Aucun déphasage ne s'est produit après stockage pendant 6 mois à la température ambiante.

### EXEMPLE 3 : EMULSION POUR USAGE EXTERNE

De la même manière que dans l'exemple 1, on a préparé une émulsion contenant:

| | |
|---|---|
| Alcool cétéarylique/ cétéarylique sulfate de sodium/ Laurylsulfate de sodium (ratio en poids: 60%/35%/5%) | 0,05g |
| Carbonate de dicaprylyl | 0,011g |
| Propylparabene/ benzoate parabene (50%/50% p/p) | 0,004g |
| Eau purifiée | qsp 1g |
| Glycérine | 0,01g |
| Kaolinite | 0,1g |
| Gomme Xanthane | 0,01g |
| Agent chélatant amphotère | 0,002g |

Cette émulsion huile dans l'eau présente les caractéristiques suivantes:
- viscosité Brookfield de 14500 cP, mesurée à une température de 20(+/-1)°C et avec une vitesse de mobile à 3/20 rpm
- pH de 6,80
- Aucun déphasage ne s'est produit après stockage pendant 6 mois à la température ambiante.

### EXEMPLE 4 : EMULSION POUR USAGE EXTERNE

De la même manière que dans l'exemple 1, on a préparé une émulsion contenant:

| | |
|---|---|
| Eau purifiée | qsp 1g |
| isononyl isononanoate | 0,045g |
| Octyl dodécanol et octyldodécyl xyloside et PEG-30 Dipolyhydroxystéarate | 0,025g |
| Polyacrylate-13 et Polyisobutylène et polysorbate-20 | 0,01g |
| Glycérine | 0,03g |
| Sesquichlorhydrate d'aluminium | 0,15g |
| Benzoate de sodium (et) sorbate de potassium | 0,0045g |
| Agent chélatant amphotère | 0,002g |

Cette émulsion huile dans l'eau présente les caractéristiques suivantes:
- viscosité Brookfield de 12000 cP, mesurée à une température de 20(+/-1)°C et avec une vitesse de mobile à 3/20 rpm
- pH de 4,30
- Aucun déphasage ne s'est produit après stockage pendant 6 mois à la température ambiante.

### EXEMPLE 5: ESSAI D'EFFICACITE CONTRE DES IRRITANTS

On prépare des explants de peau d'un diamètre moyen de 10 mm, provenant d'une abdoplastie d'une femme caucasienne de 49 ans. Ces explants sont maintenus vivants dans un milieu de culture BEM (BIO-EC explant Médium) à 37°C en atmosphère humide et enrichie de 5% de CO₂.

On teste l'efficacité de la crème contre les irritants suivants :
Les irritants en solution aqueuse:
   - Hydroxyde de sodium à pH 11,50 - Ref : S/4945/PB15 Fisher Chemical;
   - Acide chlorhydrique à pH 2,00 - Ref: H/1000/PB18 Fisher Chemical;
   - Hypochlorite de sodium à 12% - Ref: 27895 Prolabo;
   - Laurylsulfate de sodium à 5% w/w - Cognis
Les irritants solubles dans huile:
   - Huile minérale - Paraffine Liquide Codex Interchimie;
   - White spirit (C9-C16 Alkane/cycloalkane) -Shellsol D60, Shell.

A T₀, on applique la crème de l'exemple 1 ci-dessus sur les explants de peau à un dosage de 3 mg/cm². On laisse sécher pendant 15 mn. Ensuite, 25µl d'une solution d'un irritant choisi dans la liste ci-dessus sont appliqués sur les explants de peau protégés (test) et sont également appliqués sur des explants de peau non-protégés (témoin). On laisse reposer jusqu'à T₄ₕ, ce qui représente 4 heures d'exposition aux irritants.

Parallèlement on prépare de la même façon des explants protégés par la crème mais qui ne sont pas mis en contact avec une substance à tester (contrôle).

L'efficacité de protection contre des irritants est mesurée par l'analyse de morphologie cellulaire après coloration au trichrome de Masson, variante Goldner. La présence de modifications morphologiques signale que la substance testée a suscité une irritation forte qui a ainsi modifié la structure des cellules.

Des échantillons sont prélevés pour tous les explants à T₀ et à T₄ₕ et sont photographiés.

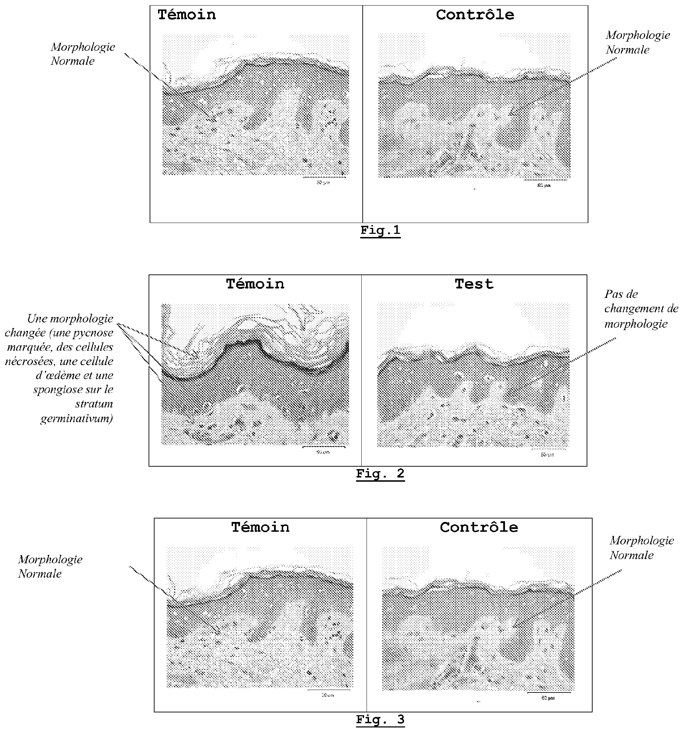

Les résultats sont représentés sur les Figures 1 et 2 pour la solution d'hydroxyde de sodium à pH 11,5(+/-0,1). La figure 1 représente les explants à Tₒ et la figure 2 à T₄ₕ.

A T₀, le stratum corneum est fin,
modérément compact et modérément kératinisé en surface, un peu plus en profondeur.
• L'épiderme présente de 4 à 5 couches cellulaires avec une bonne morphologie et une spongiose légère dans les couches basales. Le relief de jonction entre le derme et l'épiderme est net. Le derme papillaire présente un réseau dense de fibres collagène assez épaisse. Il est bien cellularisé sans zone de lyse histologique apparente.

A T₄ₕ l'épiderme des explants non-protégés présente 4-5 couches cellulaires avec de nets changements morphologiques. Ces changements sont caractérisés par une importante dénaturation cytoplasmique, une pycnose nucléaire et une nette spongiose dans le stratum germinativum.

En revanche, les explants protégés ("test") et "contrôle" ne montrent aucun changement histologique significatif.

Des résultats tout à fait analogues sont observés avec les autres irritants testés.

L'absence ou le bas niveau de changement cellulaire ainsi que la concentration faible d'interleukines ont démontré que l'émulsion huile dans l'eau de l'exemple 1 protège la peau efficacement contre les irritants testés.

### EXEMPLE 6 : ESSAI D'EFFICACITE CONTRE DES ALLERGENES

On prépare des explants comme décrit à l'exemple 5 ci-dessus.

On teste l'efficacité de la crème contre les allergènes suivants :
- métaux lourds (Al, As, Cd, Cr, Hg, Ni, Pb, Sr, Te) - ECP multi-element standard V Ref 0C467028 Merck;
- Disulfure de tétraméthylthiuram - T2 420 ;
- 2-Mercaptobenzothiazole - M3301;
- Carbamate de Benzyle - B18200;
- Bisphénol - 13302;
- Acrylate d'éthyle - W241806;
- Araldite 506 epoxy Resin - A3183;
- Hévéine - latex 4335932 ;
- Méthanal à 30% w/w - 116 99031.

A T₀, on applique la crème de l'exemple 2 ci-dessus sur les explants de peau à un dosage de 3 mg/cm². On laisse sécher pendant 15 mn. Ensuite, 25µl d'une solution de l'un des allergènes choisis dans la liste ci-dessus sont appliqués sur les explants de peau protégés par la crème (test) et sont également appliqués sur des explants de peau non-protégés (à blanc). On laisse reposer jusqu'à T₄ₕ, ce qui représente 4 heures d'exposition aux allergènes sans lavage ni friction abrasive.

Parallèlement on prépare de la même façon des explants protégés par la crème mais qui ne sont pas mis en contact avec une substance à tester (contrôle).

L'efficacité de protection contre des allergènes est mesurée par l'analyse d'immuno-coloration des récepteurs CD1a des cellules de Langerhans. D'abord, les sections paraffinées de cellules Langerhans sont immuno-colorées avec des anticorps monoclonaux anti-CD1a (ref. IM1590, clone 010, Beckman Coulter) pendant 1 heure à température ambiante. Cette immuno-coloration est renforcée par un système de streptavidine/biotine (Vector, PK-7200) et révélée en employant VIP (Vector, SK-4600). Les nucléus sont contre-colorés par hémalum de Masson. Les cellules Langerhans sont comptées sur chaque section le long d'épiderme. La longueur de chaque section est mesurée par le logiciel Olympus Cell et le nombre moyen de cellules Langerhans par centimètre d'épiderme est calculé.

L'efficacité de protection contre des allergènes est mesurée par l'analyse d'immuno-coloration de CD1a. D'abord, les sections paraffinées de cellules Langerhans sont immuno-colorées avec des anticorps monoclonaux anti-CD1a (ref. IM1590, clone O10, Beckman Coulter) pendant 1 heure à température ambiante. Cette immuno-coloration est renforcée par un système de streptavidine/biotine (Vector, PK-7200) et révélée en employant VIP (Vector, SK-4600). Les nucléus sont contre-colorés par hémalum de Masson. Les cellules Langerhans sont comptées sur chaque section le long d'épiderme. La longueur de chaque section est mesurée par le logiciel Olympus Cell et le nombre moyen de cellules Langerhans par centimètre d'épiderme est calculé.

L'efficacité de protection contre l'allergène est mesurée par le nombre de cellules Langerhans n'ayant pas migré.

Des échantillons sont prélevés pour tous les explants à T₀ et à T₄ₕ et sont microphotographiés.

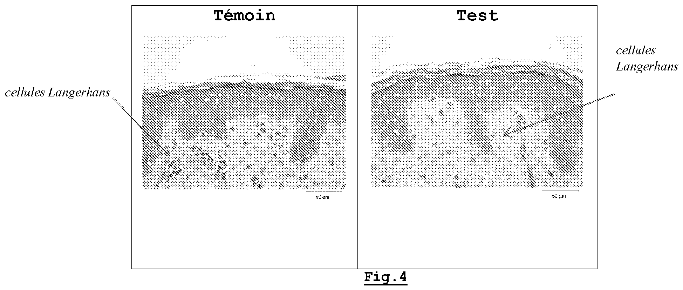

Les résultats sont présentés sur les figures 3 et 4. La figure 3 représente les explants à Tₒ et la figure 4 à T₄ₕ pour l'Hévéine.

A T₀, le stratum corneum est fin, compact et modérément rempli de kératine en surface et remarquablement en bas. L'épiderme présente de 4 à 5 couches cellulaires avec une bonne morphologie et une spongiose légère sur le stratum germinativum. Le relief de jonction entre le derme et l'épiderme est marqué. Le derme papillaire présente des fibres assez épaisses construisant un réseau dense et il est bien cellularisé sans zone de lyse histologique apparente.

L'épiderme des explants non-protégés présente 4-5 couches cellulaires avec des changements morphologiques remarquables. Ces changements sont caractérisés par une dénaturation cytoplasmique marquée (altération protéique), une pycnose marquée et une spongiose marquée sur le stratum germinativum.

En revanche, les explants protégés et "contrôle" ne montrent aucun changement histologique significatif.

Des résultats analogues ont été obtenus avec chacun des autres allergènes testés.

L'absence ou le bas niveau de changement cellulaire ainsi que la concentration faible d'interleukines ont démontré que la crème de l'exemple 2 protège la peau efficacement contre les allergènes testés.

## Revendications

1. Emulsion comprenant au moins un agent chélatant amphotère qui comprend un complexe à base d'aluminium et d'acide éthylène diamine tétraacétique ou son sel trisodique présentant la formule générale [Al(Y)Bₙ]^{c'}D_{c} avec B représentant OH⁻, BO₂⁻, H⁺, Y représentant un tétracarboxylate pouvant être protoné quatre fois pour former l'acide éthylène diamine tétraacétique, n représentant un nombre entier égal à 0, 1, 2 ou 3, D étant un contre-ion, de préférence Na⁺, c étant un nombre entier égal à 0, 1, 2 ou 3 et c' étant un nombre relatif ayant la même valeur absolue que c ; **caractérisée par le fait que** ledit agent chélatant amphotère est stabilisé par un acide aminé choisi dans le groupe comprenant la glycine, l'histidine, l'arginine, la lysine, la phénylalanine, l'alanine, l'isoleucine, la leucine, la méthionine, la proline, la valine, le tryptophane, la sérine, la glutamine, la cystine et leurs mélanges,
pour son utilisation pour protéger la peau contre des agents chimiques agressifs, ladite émulsion étant appliquée au moins 5 minutes, de préférence au moins 10 minutes et plus préférentiellement encore au moins 15 minutes avant le contact avec ledit agent chimique agressif.

2. Emulsion pour son utilisation selon la revendication 1, **caractérisée par le fait que** l'agent chimique agressif est choisi dans les catégories H311, H312, H314, H315, H317 du règlement CLP (règlement (CE) n°1272/2008), les produits ayant un pH de 2 à 11,5 et leurs mélanges.

3. Emulsion pour son utilisation selon la revendication 1 ou 2, pour protéger la peau contre des allergènes.

4. Emulsion pour son utilisation selon la revendication 3, **caractérisée par le fait que** l'allergène est choisi dans le groupe comprenant des métaux lourds tels que Al, As, Cd, Cr, Hg, Ni, Pb, Sr et Te, le disulfure de tétraméthylthiuram, le 2-mercaptobenzothiazole, le carbamate de benzyle, le bisphénol, l'acrylate d'éthyle, l'araldite 506 epoxy Resin, l'hévéine, le méthanal et leurs mélanges.

5. Emulsion comprenant au moins un agent chélatant amphotère qui comprend un complexe à base d'aluminium et d'acide éthylène diamine tétraacétique ou son sel trisodique présentant la formule générale [Al(Y)Bₙ]^{c'}D_{c} avec B représentant OH⁻, BO₂⁻, H⁺, Y représentant un tétracarboxylate pouvant être protoné quatre fois pour former l'acide éthylène diamine tétraacétique, n représentant un nombre entier égal à 0, 1, 2 ou 3, D étant un contre-ion, de préférence Na⁺, c étant un nombre entier égal à 0, 1, 2 ou 3 et c' étant un nombre relatif ayant la même valeur absolue que c ; ledit agent chelant amphotère étant stabilisé par un acide aminé choisi dans le groupe comprenant la glycine, l'histidine, l'arginine, la lysine, la phénylalanine, l'alanine, l'isoleucine, la leucine, la méthionine, la proline, la valine, le tryptophane, la sérine, la glutamine, la cystine et leurs mélanges, pour une utilisation dans la prévention des dermites de contact irritatives et des dermites de contact allergiques où les dermites de contact allergiques sont des réactions de type retardé.

6. Emulsion pour une utilisation selon l'une quelconque des revendications 1 à 5, **caractérisée par le fait que** l'émulsion est appliquée régulièrement sur toute la surface, à raison d'environ 0,5 à 5 mg/cm², de préférence d'environ 1 à 3 mg/cm², et plus préférentiellement encore d'environ 2 mg/cm².

7. Emulsion pour une utilisation selon la revendication 5 ou 6, **caractérisée par le fait que** l'émulsion est appliquée au moins 5 minutes, de préférence au moins 10 minutes et plus préférentiellement encore au moins 15 minutes avant le contact avec un allergène.

8. Emulsion pour une utilisation selon l'une quelconque des revendications 1 à 7, **caractérisée par le fait que** l'application de l'émulsion est renouvelée au plus toutes les 4 heures, de préférence à chaque lavage de mains.

9. Emulsion pour une utilisation selon l'une quelconque des revendications 1 à 8, **caractérisée par le fait que** la concentration en agent chélatant amphotère est de 0,01 à 5% en poids du poids total de l'émulsion.

10. Emulsion pour une utilisation selon l'une quelconque des revendications 1 à 9, **caractérisée par le fait que** l'émulsion comprend au moins un agent tensio-actif qui peut être hydrophobe avec un HLB de 3 à 10 ou hydrophile avec un HLB de 11 à 18.

11. Emulsion pour une utilisation selon l'une quelconque des revendications 1 à 10, **caractérisée par le fait que** la concentration en agent tensio-actif est de 0,1% à 10% en poids, de préférence de 0,5 à 5% en poids, et plus préférentiellement encore de 1 à 3% en poids du poids total de l'émulsion.

12. Emulsion pour une utilisation selon l'une quelconque des revendications 1 à 11, **caractérisée par le fait que** ladite émulsion est une émulsion huile dans l'eau.

## Patentansprüche

1. Emulsion, welche wenigstens ein amphoteres chelatbildendes Mittel umfasst, das einen Komplex auf der Basis von Aluminium und Ethylen-Diamintetraessigsäure oder ihrem Trinatriumsalz mit der allgemeinen Formel [Al(Y)Bn]^{c'}D_{c} aufweist, wobei B für OH⁻, BO₂⁻, H⁺ steht; Y für ein Tetracarboxylat steht, das viermal protoniert werden kann, um Ethylen-Diamintetraessigsäure zu bilden, n für eine ganze Zahl gleich 0, 1, 2 oder 3 steht, D ein Gegenion ist, bevorzugt Na⁺, c für eine ganze Zahl gleich 0, 1, 2 oder 3 steht und c' ein Relativwert mit dem gleichen absoluten Wert wie c ist; **dadurch gekennzeichnet, dass** das amphotere chelatbildende Mittel durch eine Aminosäure stabilisiert ist, die gewählt ist aus der Gruppe, umfassend Glycin, Histidin, Arginin, Lysin, Phenylalanin, Alanin, Isoleucin, Leucin, Methionin, Prolin, Valin, Tryptophan, Serin, Glutamin, Cystin und deren Mischungen,
wobei zur Anwendung zum Schutz der Haut gegen agressive chemische Mittel die Emulsion wenigstens 5 Minuten, bevorzugt wenigstens 10 Minuten und noch stärker bevorzugt wenigstens 15 Minuten vor dem Kontakt mit dem agressiven chemischen Mittel aufgetragen wird.

2. Emulsion zur Anwendung nach Anspruch 1, **dadurch gekennzeichnet, dass** das agressive chemische Mittel aus den Kategorien H311, H312, H314, H315, H317 der CLP-Verordnung (Verordnung (EU) Nr. 1272/2008) gewählt ist, wobei die Produkte einen pH-Wert von 2 bis 11,5 aufweisen, sowie deren Mischungen.

3. Emulsion zur Anwendung nach Anspruch 1 oder 2 zum Schutz der Haut gegen Allergene.

4. Emulsion zur Anwendung nach Anspruch 3, **dadurch gekennzeichnet, dass** das Allergen aus der Gruppe gewählt ist, welche Schwermetalle wie zum Beispiel Al, As, Cd, Cr, Hg, Ni, Pb, Sr und Te, Tetramethylthiuram-Disulfid, 2-Mercaptobenzothiazol, Benzylcarbamat, Bisphenol, Ethylacrylat, Epoxyharz Araldit 506, Hevein, Methanal und deren Mischungen umfasst.

5. Emulsion, welche wenigstens ein amphoteres chelatbildendes Mittel umfasst, das einen Komplex auf der Basis von Aluminium und Ethylen-Diamintetraessigsäure oder ihrem Trinatriumsalz mit der allgemeinen Formel [Al(Y)Bn]^{c'}D_{c} aufweist, wobei B für OH⁻, BO₂⁻, H⁺ steht; Y für ein Tetracarboxylat steht, das viermal protoniert werden kann, um Ethylen-Diamintetraessigsäure zu bilden, n für eine ganze Zahl gleich 0, 1, 2 oder 3 steht, D ein Gegenion ist, bevorzugt Na⁺, c für eine ganze Zahl gleich 0, 1, 2 oder 3 steht und c' ein Relativwert mit dem gleichen absoluten Wert wie c ist; **dadurch gekennzeichnet, dass** das amphotere chelatbildende Mittel durch eine Aminosäure stabilisiert ist, die gewählt ist aus der Gruppe, umfassend Glycin, Histidin, Arginin, Lysin, Phenylalanin, Alanin, Isoleucin, Leucin, Methionin, Prolin, Valin, Tryptophan, Serin, Glutamin, Cystin und deren Mischungen,
zur Anwendung zur Prävention von irritativen Kontaktdermatitiden und allergischen Kontaktdermatitiden, wobei die allergischen Kontaktdermatitiden Reaktionen vom verzögerten Typ sind.

6. Emulsion zur Anwendung nach einem der Ansprüche 1 bis 5, **dadurch gekennzeichnet, dass** die Emulsion regelmäßig auf der ganzen Oberfläche verteilt wird zu ungefähr 0,5 bis 5 mg/cm², bevorzugt ungefähr 1 bis 3 mg/cm² und noch stärker bevorzugt ungefähr 2 mg/cm².

7. Emulsion zur Anwendung nach Anspruch 5 oder 6, **dadurch gekennzeichnet, dass** die Emulsion wenigstens 5 Minuten, bevorzugt wenigstens 10 Minuten und noch stärker bevorzugt wenigstens 15 Minuten vor dem Kontakt mit einem Allergen aufgetragen wird.

8. Emulsion zur Anwendung nach einem der Ansprüche 1 bis 7, **dadurch gekennzeichnet, dass** die Anwendung der Emulsion höchstens alle 4 Stunden wiederholt wird, bevorzugt nach jedem Händewaschen.

9. Emulsion zur Anwendung nach einem der Ansprüche 1 bis 8, **dadurch gekennzeichnet, dass** die Konzentration des amphoteren chelatbildenden Mittels 0,01 bis 5% Gewichtsprozent des Gesamtgewichts der Emulsion beträgt.

10. Emulsion zur Anwendung nach einem der Ansprüche 1 bis 9, **dadurch gekennzeichnet, dass** die Emulsion wenigstens ein oberflächenaktives Mittel umfasst, das hydrophob sein kann, mit einem HLB von 3 bis 10 oder hydrophil mit einem HLB von 11 bis 18.

11. Emulsion zur Anwendung nach einem der Ansprüche 1 bis 10, **dadurch gekennzeichnet, dass** die Konzentration des oberflächenaktiven Mittels 0,1 bis 10 Gewichtsprozent beträgt, bevorzugt 0,5 bis 5 Gewichtsprozent und noch stärker bevorzugt 1 bis 3 Gewichtsprozent des Gesamtgewichts der Emulsion.

12. Emulsion zur Anwendung nach einem der Ansprüche 1 bis 11, **dadurch gekennzeichnet, dass** die Emulsion eine Öl-in-Wasser Emulsion ist.

## Claims

1. Emulsion comprising at least one amphoteric chelating agent which comprises a complex based on aluminium and ethylene diamine tetraacetic acid or the trisodium salt thereof having the general formula [Al(Y)Bₙ]^{c'}D_{c} where B represents OH⁻, BO₂⁻, H⁺, Y represents a tetracarboxylate optionally protonated four times to form ethylene diamine tetraacetic acid, n represents an integer equal to 0, 1, 2 or 3, D is a counterion, preferably Na⁺, c is an integer equal to 0, 1, 2 or 3, and c' is a relative number having the same absolute value as c; **characterised in that** said amphoteric chelating agent is stabilised with an amino acid chosen in the group comprising glycine, histidine, arginine, lysine, phenylalanine, alanine, isoleucine, leucine, methionine, proline, valine, tryptophan, serine, glutamine, cystine and mixtures thereof,
for use thereof for protecting the skin against aggressive chemical agents, said emulsion being applied at least 5 minutes, preferably at least 10 minutes and more preferentially at least 15 minutes prior to contact with said aggressive chemical agent.

2. Emulsion for use thereof according to claim, 1, **characterised in that** the aggressive chemical agent is chosen in the categories H311, H312, H314, H315, H317 of the CLP regulation (regulation (EC) No. 1272/2008), substances having a pH of 2 to 11.5 and mixtures thereof.

3. Emulsion for use thereof according to claim 1 or 2, for protecting the skin against allergens.

4. Emulsion for use thereof according to claim 3, **characterised in that** the allergen is chosen in the group comprising heavy metals such as Al, As, Cd, Cr, Hg, Ni, Pb, Sr and Te, tetramethylthiuram disulphide, 2-mercaptobenzothiazole, benzyl carbamate, bisphenol, ethyl acrylate, araldite 506 epoxy resin, hevein, methanal and mixtures thereof.

5. Emulsion comprising at least one amphoteric chelating agent which comprises a complex based on aluminium and ethylene diamine tetraacetic acid or the trisodium salt thereof having the general formula [Al(Y)Bₙ]^{c'}D_{c} where B represents OH⁻, BO₂⁻, H⁺, Y represents a tetracarboxylate optionally protonated four times to form ethylene diamine tetraacetic acid, n represents an integer equal to 0, 1, 2 or 3, D is a counterion, preferably Na⁺, c is an integer equal to 0, 1, 2 or 3, and c' is a relative number having the same absolute value as c; said amphoteric chelating agent being stabilised with an amino acid chosen in the group comprising glycine, histidine, arginine, lysine, phenylalanine, alanine, isoleucine, leucine, methionine, proline, valine, tryptophan, serine, glutamine, cystine and mixtures thereof, for use thereof in the prevention of irritant contact dermatitis and allergic contact dermatitis where the allergic contact dermatitis are delayed type reactions.

6. Emulsion for use according to any one of claims 1 to 5, **characterised in that** the emulsion is applied regularly on the entire surface area, at a rate of approximately 0.5 to 5 mg/cm², preferably approximately 1 to 3 mg/cm², and more preferentially approximately 2 mg/cm².

7. Emulsion for use according to claim 5 or 6, **characterised in that** the emulsion is applied at least 5 minutes, preferably at least 10 minutes and more preferentially at least 15 minutes prior to contact with an allergen.

8. Emulsion for use according to any one of claims 1 to 7, **characterised in that** the application of the emulsion is repeated at most every 4 hours, preferably each time the hands are washed.

9. Emulsion for use according to any one of claims 1 to 8, **characterised in that** the amphoteric chelating agent is from 0.01 to 5% by weight of the total weight of the emulsion.

10. Emulsion for use according to any one of claims 1 to 9, **characterised in that** the emulsion comprises at least one surfactant which may be hydrophobic with an HLB of 3 to 10 or hydrophilic with an HLB of 11 to 18.

11. Emulsion for use according to any one of claims 1 to 10, **characterised in that** the surfactant concentration is from 0.1% to 10% by weight, preferably from 0.5 to 5% by weight, and more preferentially from 1 to 3% by weight of the total weight of the emulsion.

12. Emulsion for use according to any one of claims 1 to 11, **characterised in that** said emulsion is an oil-in-water emulsion.
